# EUROPEAN PATENT SPECIFICATION

(11) **EP 2 662 691 B1**
(45) Date of publication and mention of the grant of the patent: **11.10.2017**
(21) Application number: 13164630.9
(22) Date of filing: 22.04.2013
(51) Int. Cl.: G01N 33/50, G01N 33/68

(54) **Basophil and high affinity IgE receptor materials and methods relating to the diagnosis of anaphylaxis**
Basophile und hochaffine IgE-Rezeptor-Materialien und Verfahren im Zusammenhang mit der Diagnose von Anaphylaxie
Basophiles et matériaux du récepteur IgE à haute affinité et procédés relatifs au diagnostic de l'anaphylaxie

(30) Priority: 07.05.2012 SI 201200140
(43) Date of publication of application: 13.11.2013
(73) Proprietor: University Clinic of Respiratory and Allergic Diseases Golnik, 4204 Golnik (SI)
(72) Inventor: Korosec, Peter, 1000 Ljubljana (SI); Kosnik, Mitja, 4000 Kranj (SI); Silar, Mira, 4000 Kranj (SI)
(74) Representative: Kalhammer, Georg

(56) References cited:
- WO-A2-95/15376
- SHELLEY, W.B. AND PARNES, H. M.: "The Absolute Basophil Count", JAMA, vol. 192, no. 5, 3 May 1965 (1965-05-03), pages 108-110, XP009171605,
- M. KOSNIK ET AL: "High sensitivity of basophils predicts side-effects in venom immunotherapy", ALLERGY, vol. 60, no. 11, 1 November 2005 (2005-11-01), pages 1401-1406, XP055073453, ISSN: 0105-4538, DOI: 10.1111/j.1398-9995.2005.00894.x
- O. V. HAUSMANN ET AL: "Robust expression of CCR3 as a single basophil selection marker in flow cytometry", ALLERGY, vol. 66, no. 1, 3 January 2011 (2011-01-03), pages 85-91, XP055073456, ISSN: 0105-4538, DOI: 10.1111/j.1398-9995.2010.02431.x
- EVA UNTERSMAYR ET AL: "The High Affinity IgE Receptor Fc[epsilon]RI Is Expressed by Human Intestinal Epithelial Cells", PLOS ONE, vol. 5, no. 2, E9023, 1 January 2010 (2010-01-01), pages 1-11, XP055073465, ISSN: 1932-6203, DOI: 10.1371/journal.pone.0009023
- DOMBROWICZ D ET AL: "Abolition of anaphylaxis by targeted disruption of the high affinity immunoglobulin E receptor alpha chain gene", CELL, CELL PRESS, US, vol. 75, no. 5, 3 December 1993 (1993-12-03), pages 969-976, XP024246141, ISSN: 0092-8674, DOI: 10.1016/0092-8674(93)90540-7 [retrieved on 1993-12-03]
- SIHRA ET AL: "Expression of high-affinity IgE receptors (FcepsilonRI) on peripheral blood basophils, monocytes, and eosinophils in atopic and nonatopic subjects: Relationship to total serum IgE concentrations", JOURNAL OF ALLERGY AND CLINICAL IMMUNOLOGY, ELSEVIER, AMSTERDAM, NL, vol. 99, no. 5, 1 May 1997 (1997-05-01), pages 699-706, XP005189300, ISSN: 0091-6749, DOI: 10.1016/S0091-6749(97)70033-2
- BÉNÉ MC1 ET AL: "Automated cell count in flow cytometry: a valuable tool to assess CD4 absolute levels in peripheral blood", AMERICAN JOURNAL OF CLINICAL PATHOLOGY, AMERICAN SOCIETY FOR CLINICAL PATHOLOGY, US, vol. 110, no. 3, 1 September 1998 (1998-09-01), pages 321-326, XP009183448, ISSN: 0002-9173
- M. V. Khodoun ET AL: "Identification of markers that distinguish IgE- from IgG-mediated anaphylaxis", Proceedings of the National Academy of Sciences, vol. 108, no. 30, 11 July 2011 (2011-07-11), pages 12413-12418, XP055267555, US ISSN: 0027-8424, DOI: 10.1073/pnas.1105695108
- BRUHNS P ET AL: "Regulation of allergy by Fc receptors", CURRENT OPINION IN IMMUNOLOGY, ELSEVIER, OXFORD, GB, vol. 17, no. 6, 1 December 2005 (2005-12-01), pages 662-669, XP027787138, ISSN: 0952-7915 [retrieved on 2005-12-01]
- Yusuke Tsujimura ET AL: "Basophils Play a Pivotal Role in Immunoglobulin-G-Mediated but Not Immunoglobulin-E-Mediated Systemic Anaphylaxis", IMMUNITY., vol. 28, no. 4, 1 April 2008 (2008-04-01), pages 581-589, XP055373341, US ISSN: 1074-7613, DOI: 10.1016/j.immuni.2008.02.008
- J. Eckl-Dorna ET AL: "Basophils are not the key antigen-presenting cells in allergic patients", ALLERGY, vol. 67, no. 5, 16 February 2012 (2012-02-16), pages 601-608, XP055373343, United Kingdom ISSN: 0105-4538, DOI: 10.1111/j.1398-9995.2012.02792.x
- C. Kitzmüller ET AL: "Human blood basophils do not act as antigen-presenting cells for the major birch pollen allergen Bet v 1", ALLERGY, vol. 67, no. 5, 1 May 2012 (2012-05-01), pages 593-600, XP055373345, United Kingdom ISSN: 0105-4538, DOI: 10.1111/j.1398-9995.2011.02764.x
- Thomas A Wynn: "Basophils trump dendritic cells as APCs for TH2 responses", NATURE IMMUNOLOGY, vol. 10, no. 7, 1 July 2009 (2009-07-01), pages 679-681, XP055373346, ISSN: 1529-2908, DOI: 10.1038/ni0709-679
- J. J. Lee ET AL: "When is a mouse basophil not a basophil?", BLOOD, vol. 109, no. 3, 5 October 2006 (2006-10-05), pages 859-861, XP055373347, US ISSN: 0006-4971, DOI: 10.1182/blood-2006-06-027490
- Edward F. Knol ET AL: "Basophils and antigen presentation: of mice and not men?", ALLERGY, vol. 67, no. 5, 11 April 2012 (2012-04-11) , pages 579-580, XP055373352, United Kingdom ISSN: 0105-4538, DOI: 10.1111/j.1398-9995.2012.02816.x

## Description

The present invention relates to methods and materials for the diagnosis of anaphylaxis, and more particularly to the role of basophil absolute count and the expression of the high affinity IgE in the occurrence of anaphylaxis. Methods of diagnosing anaphylaxis by determining the absolute number of basophils in whole blood or by determining the expression of the α chain of high affinity IgE receptor in whole blood are disclosed.

### Field of the Invention

The present invention relates to materials and methods for the diagnosis of anaphylaxis, and more particularly to the role of basophil absolute count and the expression of α chain of the high affinity IgE in the occurrence of anaphylaxis.

### Background of the Invention

Anaphylaxis is a severe, systemic mainly IgE-mediated allergic reaction that may lead to death by airway obstruction or vascular collapse. Anaphylaxis may involve immunologic or non-immunologic mechanisms other than IgE, but this invention is focused on IgE mediated anaphylaxis. Anaphylaxis involves the activation of effectors cells through an IgE antibody and it is most commonly triggered by insect venom, food or medication. The diagnosis is preferably made on clinical criteria. However, the anaphylactic reaction can demonstrate high clinical variability related to the target organ affected, allergen threshold, response to therapy and thus laboratory test to confirm the clinical diagnosis is often needed. The most widely used test is total tryptase measurement in serum or plasma. Although an elevated level might supports the diagnosis of anaphylaxis, the tryptase levels are often within normal limits. Blood histamine level is also elevated early during reaction, but very quickly returns to baseline, and thus this test is impractical in clinical setting. Therefore, better laboratory tests with increased sensitivity and/or practicality are needed to improve recognition of the disease, and implement of risk reduction measures.

Shelley and Parnes (1965) JAMA 192(5), pages 108-110 describes the technique and significance of the absolute basophil count.

Kosnik et al. (2005) Allergy 60, pages 1401-1406 describes studies which suggest that increased basophil sensitivity to allergen-specific *in vitro* stimulation is associated with side-effects in venom immunotherapy.

Hausmann et al. (2011) Allergy 66, pages 85-91 investigates whether robust expression of CCR3 is a single basophil selection marker in flow cytometry.

Untersmayr et al. (2010) PLoS ONE 5(2) E9023, pages 1-11 reports that the high affinity IgE receptor FcεRI is expressed by human intestinal epithelial cells.

Dombrowicz et al. (1993) Cell 75(3), pages 969-976 describes experiments suggesting that FcεRI is necessary for the initiation of IgE-dependent anaphylactic reactions.

WO 95/15376 A2 describes *in vivo* assays for inhibitors of IgE-mediated allergic responses.

Sihra et al. (1997) Journal of Allergy and Clinical Immunology 99(5), pages 699-706 investigates the relationship of FcεRI expression to total serum IgE concentrations.

Béné et al. (1998) American Journal of Clinical Pathology 110(3), pages 321-326 describes tools and methods for determining absolute levels of lymphocytes.

### Summary of the Invention

The present invention is defined in the claims.

The present invention drives from investigations to determine whether there is a correlation between anaphylaxis and changes in absolute number of basophils in whole blood and the changes in expression of α chain of the high affinity IgE receptor in whole blood.

In order to gain information on the significance of the disordered absolute number of basophils and of the disordered expression of the high affinity IgE receptor in whole blood in anaphylaxis, comparison has been made in which absolute number of basophils and the expression of the α chain of high affinity IgE receptor was measured after anaphylaxis.

Accordingly, in a first aspect, the present disclosure provides the use of absolute basophils count in whole blood for diagnosis of anaphylaxis.

In further aspect, the present disclosure provides the use of expression of the α chain of high affinity IgE receptor in whole blood for diagnosis of anaphylaxis.

The present invention provides a method for the diagnosis of anaphylaxis with the use of a basophil absolute count, the method comprising the steps of:
(1) measuring an absolute number of basophils in a biological sample of whole blood, which is obtained from a patient up to 24h after suspected anaphylaxis episode; wherein the basophils are marked with antibody capable of specifically binding the basophils and analyzed by flow cytometry and wherein the absolute count is obtained by microbeads and
(2) diagnosing that the patient has experienced anaphylaxis when the number of basophils measured in Step (1) is lower than a corresponding cut-off point measured before or later after the anaphylaxis.

In further aspect, the present invention provides a method for the diagnosis of anaphylaxis with the use of expression of the α chain of high affinity IgE receptor, the method comprising the steps of:
(1) measuring an expression level of α chain of high affinity IgE receptor in a biological sample of whole blood, which is obtained from a patient up to 24h after suspected anaphylaxis episode; and
(2) diagnosing that the patient has experienced anaphylaxis when the expression level measured in Step (1) is lower than a corresponding cut-off point measured before or later after the anaphylaxis.

Some of the methodological properties of absolute basophils count and α chain of high affinity IgE receptor assays to determine the properties that can be used in the diagnosis of anaphylaxis are set out in the description below. This allows those of ordinary skill in the art to use of this methodology for the diagnosis of anaphylaxis.

The results of our investigations described below indicate the following:
(a) The absolute number of whole blood basophils is significantly lower after anaphylaxis (2 -to 200-fold) than 7 days or 1 month after anaphylaxis and also lower than predetermined cut-off point measured before anaphylaxis or in matched control subjects.
(b) The expression of the α chain of high affinity IgE receptor in whole blood is significantly lower after anaphylaxis (at least 1.5-fold) than 7 days or 1 month after anaphylaxis and also lower than predetermined cut-off point possibly measured before anaphylaxis or in matched control subjects.
(c) The decrease in absolute number of whole blood basophils and the decrease in expression of the α chain of high affinity IgE receptor in whole blood was comparably high between subjects with different severity of anaphylactic reaction (Mueller grade I, Mueller grade II, Mueller grade III and Mueller grade IV).
(d) The decrease in absolute number of whole blood basophils and the decrease in expression of the α chain of high affinity IgE receptor in whole blood was comparably high between patients in which anaphylaxis was triggered by Hymenoptera insect venom, food or medication.

The present disclosure provides a method of diagnosing of anaphylaxis in a patient, the method comprising determining the absolute number of basophils and expression of α chain of high affinity IgE receptor in a biological sample obtained from the patient. Thus, a diagnosis can then be made by observation of the decrease of absolute number of basophils and the decrease of expression of α chain of high affinity IgE receptor after anaphylaxis.

The present invention will now be described by way of example and not limitation with reference to the accompanying drawings.

### Brief Description of the Drawings

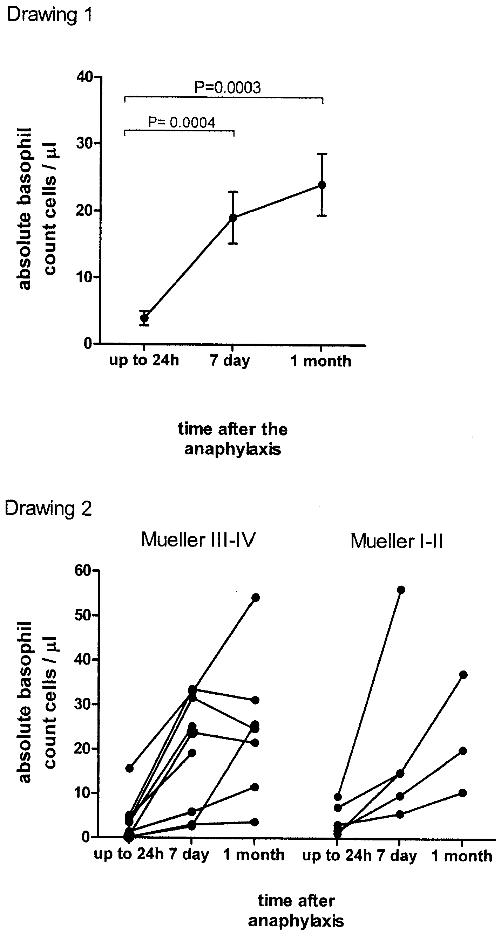

Drawing 1 shows the effect of anaphylaxis on absolute number of basophils in whole blood (cells/µl) in a time periods up to 24 hours after anaphylaxis, 7 days after anaphylaxis and one month after anaphylaxis.
Drawing 2 shows individual values for absolute number of basophils in whole blood in a time periods up to 24 hours after anaphylaxis, 7 days after anaphylaxis and one month after anaphylaxis. The patients are divided according the severity of anaphylaxis in patients which experienced Mueller grade I or II reactions and patients which experienced Mueller grade III or IV reactions.

### Detailed Description of the Invention

### Basophils

Basophils appear as mononuclear cells in human blood, measuring 10-14 µm in diameter, with a lobulated nucleus and round, basophilic granules in the cytoplasm. Basophils express a variety of membrane receptors through which they can respond to exogenous stimuli and the most important is high affinity IgE receptor crosslinking by IgE/allergen complexes. It is well established that basophils, exert effector functions during allergic responses and cause most of the typical clinical symptoms including anaphylactic reaction through the numerous compounds produced and released upon IgE crosslinking during the reaction following allergen exposure. The comprehensive way to analyze basophils is a flow cytometry with basophil selection markers, IgE, CD123pos/HLA-DRneg or CCR3 (eotaxin receptor) (Kosnik M, Silar M, Bajrovic N, Music E, Korosec P. High sensitivity of basophils predicts side-effects in venom immunotherapy. Allergy. 2005 Nov; 60 (11): 1401-6; Hausmann OV, Gentinetta T, Fux M, Ducrest S, Pichler WJ, Dahinden CA. Robust expression of CCR3 as a single basophil selection marker in flow cytometry. Allergy. 2011 Jan; 66 (1): 85-91). The IgE, CD123/HLA-DR or CCR3 antagonists are specific binding proteins, the antibodies. The production of polyclonal and monoclonal antibodies is well established in the art. The antibodies described are employed in the diagnostic aspects of the invention by tagging them with a label which can directly generate detectable, and preferably measurable, signals. One favored mode is by covalent linkage of each antibody with an individual fluorochrome, with spectrally isolated emission characteristics. Further repertoire includes standard flow cytometry grade fluorescent particles (microbeads) (Béné MC, Kolopp Sarda MN, El Kaissouni J, De March Kennel A, Molé C, Kohler C,Faure GC. Automated cell count in flow cytometry: a valuable tool to assess CD4 absolute levels in peripheral blood. Am J Clin Pathol. 1998 Sep; 110 (3): 321-6). This should be reference particles with known number of particles per mL or tube for counting the absolute cell number by flow cytometry.
1. Label basophils in whole blood sample by anti-IgE, CD123/HLA-DR or CCR3 antibodies and add microbeads.
2. Analyze sample on flow cytometry. Gate the basophils according to antibody labeling and gate the microbeads. Obtain the amount of events in basophil gate or region and amount of events in gate or region for the microbeads and then calculate number of basophils per µl of whole blood using the following equitation: (number of events in gate or region containing basophils / number of events in microbeads gate or region) X (number of microbeds used in test / volume of the test) = number of basophils per µl of whole blood.

### High affinity IgE receptor

The high affinity IgE receptor (FcεRI) exists in two forms. It can be expressed as a trimer or tetramer comprised of an IgE-binding α chain, a membrane tetraspanning β chain that is absent in the trimeric receptor, and a disulfide-linked homodimer of γ chains (MacGlashan D Jr. IgE and FcεRI regulation. Ann N Y Acad Sci. 2005; 1050: 73-88). While the trimeric form can be expressed on a variety of immune cells (such as monocytes, eosinophils, Langerhan cells, etc.) the tetramer is expressed primarily on mast cells which are found in different tissue and basophils which are found in blood (MacGlashan 2005). Thus the identification of α chain of high affinity IgE receptor could be relevant marker for basophils in whole blood. mRNA for α chain of high affinity IgE receptor can be detected by gene expression analysis by reverse-transcription real-time quantitative PCR and this procedure is well established in the art. Real-time quantitative PCR technology allows quantification of PCR products in "real time" during each PCR cycle, yielding a quantitative measurement of PCR products accumulated during the course of the reaction. This is most commonly achieved through the use of fluorescence based technologies or hybridization. For α chain of high affinity IgE receptor gene (*FCER1A*) expression analysis by reverse-transcription real-time quantitative PCR validated primers and probes are already developed by commercial suppliers. It will further be understood by those skilled in the art that validated primer sets speeds the experimental process and reduces unproductive effort optimizing primer sets. In our experience with reverse-transcription real-time quantitative PCR, we are able to obtain our primer and probe sets for α chain of high affinity IgE receptor from prevalidated sources.
3. Isolation of total RNA from whole blood samples.
4. The analysis of reverse transcription reaction of mRNA to cDNA and real-time PCR of gene for α chain of high affinity IgE receptor by primer and probe from prevalidated sources and by use of relative quantitation .

### Diagnosing of anaphylaxis

Anaphylaxis is a serious systemic allergic reaction that is rapid in onset and may cause death (Simons FE, Frew AJ, Ansotegui IJ, Bochner BS, Golden DB, Finkelman FD, Leung DY, Lotvall J, Marone G, Metcalfe DD, Müller U, Rosenwasser LJ, Sampson HA, Schwartz LB, van Hage M, Walls AF. Risk assessment in anaphylaxis: current and future approaches. J Allergy Clin Immunol. 2007 Jul; 120 (1 Suppl): S2-24). Critically important unmet needs in anaphylaxis risk assessment currently include lack of an optimal, readily available laboratory test to confirm the clinical diagnosis of an anaphylaxis episode. Inability to confirm the clinical diagnosis of anaphylaxis likely contributes to underrecognition and undertreatment of the disease (Simons et al. 2007). Although the clinical diagnosis can sometimes be supported by laboratory tests-for example, measurement of histamine concentrations in plasma, or of total tryptase concentrations in serum or plasma-these currently available tests have intrinsic limitations (Lin RY, Schwartz LB, Curry A, Pesola GR, Knight RJ, Lee H-S, et al. Histamine and tryptase levels in patients with acute allergic reactions: an emergency department-based study. J Allergy Clin Immunol. 2000; 106: 65-71). The blood sample must be obtained within minutes (histamine) to a few hours (tryptase) after onset of symptoms. This is impossible in the many patients who experience anaphylaxis in community settings and arrive in the emergency department some time later with resolving symptoms. Also, even when blood samples are optimally timed, tryptase levels are often within normal limits. Laboratory tests with increased sensitivity and practicality are therefore urgently needed to confirm the clinical diagnosis of anaphylaxis, improve recognition of the disease, and implement long-term risk reduction measures (Simons et al. 2007). The present invention provides methods of diagnosing IgE-mediated anaphylaxis by determining the absolute number of basohils in whole blood or by determining the expression of the α chain of high affinity IgE receptor, as defined in the claims. These inventions provide diagnostic tests for use in healthcare settings during and after immediate treatment of anaphylaxis.
5. The absolute number of whole blood basophils (cells per µl of whole blood) is significantly lower up to 24 hours after anaphylaxis (2 -to 200-fold) than 7 days or 1 month after anaphylaxis and also lower than predetermined basophil number possibly measured before anaphylaxis or in matched control subjects. This difference determines the diagnosis of anaphylaxis.
6. The relative levels of mRNA for α chain of high affinity IgE receptor isolated from whole blood are at least 1.5-fold lower up to 24 hours after anaphylaxis than 7 days or 1 month after anaphylaxis (i. e. sample up to 24 hours has 1.5-fold less mRNA of a specific gene as later samples after 7 days or 1 mont2) and also lower than predetermined expression possibly measured before anaphylaxis or in matched control subjects. This difference determines the diagnosis of anaphylaxis.

### Description of kit relevant for counting the basophil number by flow cytometry

The kit is designed for analysis of the basophil number by flow cytometry as described in proceeding steps 1 and 2 in biological sample of whole blood. The test is intended for the determination of absolute number of basophils per µl of whole blood. The principle of the assay is that the staining reagent and fluorescent particles (microbeads) are added to the biological sample, followed by lysing and washing or without washing step. Staining reagent contains monoclonal antibodies to the basophil selection markers labeled with an individual fluorochrome, with spectrally isolated emission characteristics. Microbeads are reference particles with known number of particles per mL or tube for counting the absolute cell number by flow cytometry. The flow cytometry acquisition can be performed on any flow cytometry working with a 488 nm laser diode or a 532 nm green laser diode. The analysis of the acquired data can be performed with any flow cytometry software. The number of basophils per µl of whole blood can be calculate using the following equitation: (number of events in gate or region containing basophils / number of events in microbeads gate or region) X (number of microbeads used in test / volume of the test) = number of basophils per µl of whole blood
The kit contents:
Reagents for staining (detection),
Microbeads with known number of particles,
Lysing buffer,
Wash buffer,
Instruction manual.

Description of kit relevant for expression analysis of α chain of high affinity IgE receptor The kit is designed for expression analysis of α chain of high affinity IgE receptor gene (*FCER1A*) by real time quantitative PCR as described in proceeding steps 3 and 4 in biological sample of whole blood. The test is intended for the determination of relative levels of mRNA for α chain of high affinity IgE receptor isolated from the whole blood. The principle of the assay is that the quantification of the mRNA for α chain of high affinity IgE receptor in whole blood is a relevant marker for basophils in whole blood. The criteria for total RNA preparation method are optimal RNA quality and concentration and absence of inhibitors. The criteria for reverse transcription method are high capacity of cDNA. Primers and probes for the gene of α chain of high affinity IgE receptor and the endogenous control were selected from prevalidated sources and labeled either with fluorescence or hybridization based technologies. PCR reactions are set up at default thermal conditions according primers and probes sources. All measurements are performed in triplicate for each sample and/or time point, and analyzed by use of relative quantitation.
The kit contents:
Reagents for isolation of total RNA from human cells,
Reagents for cDNA Reverse Transcription,
Primers and probes for α chain of high affinity IgE receptor gen (*FCER1A*) and endogenous control,
PCR Master Mix,
Instruction manual.

### Drawing 1

Shows the effect of anaphylaxis on absolute number of basophils in whole blood.
The results are given as Means #SEM basophils per µl of whole blood in anaphylactic group of 15 subjects.
Up to 24 hours after anaphylaxis: 3.9 (1.1) basophils per µl of whole blood
7 days after anaphylaxis: 19 (3.9) basophils per µl of whole blood
1 month after anaphylaxis: 24 (4.6) basophils per µl of whole blood
P=0.0004 and P=0.0003; paired t test.

### Drawing 2

Shows individual values for absolute number of basophils in whole blood.
The patients are divided according the severity of anaphylaxis in patients which experienced Mueller grade I or II reactions and patients which experienced Mueller grade III or IV reactions.
24 hours after anaphylaxis the concentration of basophils in whole blood was 2-fold up to 50-fold lower (median 6 fold) then 7 days after anaphylaxis.
24 hours after anaphylaxis the concentration of basophils in whole blood was 3.5-fold up to 130-fold then 1 month after anaphylaxis.

This decrease was comparably high between subjects with different severity of anaphylactic reaction (Mueller grade I, Mueller grade II, Mueller grade III and Mueller grade IV).

## Claims

1. A method for the diagnosis of anaphylaxis with the use of a basophil absolute count, the method comprising the steps of:
(1) measuring an absolute number of basophils in a biological sample of whole blood, which is obtained from a patient up to 24h after suspected anaphylaxis episode; wherein the basophils are marked with antibody capable of specifically binding the basophils and analyzed by flow cytometry and wherein the absolute count is obtained by microbeads and
(2) diagnosing that the patient has experienced anaphylaxis when the number of basophils measured in Step (1) is lower than a corresponding cut-off point measured before or later after the anaphylaxis.

2. A method for the diagnosis of anaphylaxis with the use of expression of the α chain of high affinity IgE receptor, the method comprising the steps of:
(1) measuring an expression level of α chain of high affinity IgE receptor in a biological sample of whole blood, which is obtained from a patient up to 24h after suspected anaphylaxis episode; and
(2) diagnosing that the patient has experienced anaphylaxis when the expression level measured in Step (1) is lower than a corresponding cut-off point measured before or later after the anaphylaxis.

3. The method according to claim 1 which comprises (i) labelling the basophils in the whole blood sample by anti-IgE, CD123/HLA-DR or CCR3 antibodies, (ii) adding the microbeads, and (iii) analyzing the sample by flow cytometry.

4. The method according to claim 2 wherein the mRNA for α chain of high affinity IgE receptor is detected by gene expression analysis after reverse-transcription and production of cDNA.

5. The use of a kit for *in vitro* diagnosis of anaphylaxis with the use of a basophil absolute count, said kit comprising:
- reagents for staining (detection),
- microbeads with known number of particles,
- lysing buffer,
- wash buffer, and
- instruction manual.

6. The use of a kit for *in vitro* diagnosis of anaphylaxis with the use of expression of the α chain of high affinity IgE receptor, said kit comprising:
- reagents for isolation of total RNA from human cells,
- reagents for cDNA Reverse Transcription,
- primers and probes for α chain of high affinity IgE receptor gene (*FCER1A*) and endogenous control,
- PCR Master Mix,
- instruction manual.

## Patentansprüche

1. Verfahren für die Diagnose von Anaphylaxie unter Verwendung einer Basophilenabsolutzählung, wobei das Verfahren die Schritte umfasst:
(1) Messen der absoluten Zahl der Basophilen in einer biologischen Vollblutprobe, die von einem Patienten bis zu 24 h nach einer vermeintlichen anaphylaktischen Episode erhalten wurde;
wobei die Basophilen mit Antikörper, der speziell die Basophilen binden kann, markiert werden und mittels Durchflusszytometrie analysiert werden, und wobei die Absolutzählung mittels Mikrokügelchen erhalten wird, und
(2) Diagnostizieren, dass der Patient eine Anaphylaxie erfahren hat, wenn die in Schritt (1) gemessene Anzahl der Basophilen niedriger als der entsprechende Cut-Off-Punkt ist, der vor oder nach der Anaphylaxie gemessen wurde.

2. Verfahren für die Diagnose von Anaphylaxie unter Verwendung von Expression der α-Kette des hochaffinen IgE-Rezeptors, wobei das Verfahren die Schritte umfasst:
(1) Messen des Expressionsniveaus der α-Kette des hochaffinen IgE-Rezeptors in einer biologischen Vollblutprobe, die von einem Patienten bis zu 24 h nach einer vermeintlichen anaphylaktischen Episode erhalten wurde; und
(2) Diagnostizieren, dass der Patient eine Anaphylaxie erfahren hat, wenn das in Schritt (1) gemessene Expressionsniveau niedriger als der entsprechende Cut-Off-Punkt ist, der vor oder nach der Anaphylaxie gemessen wurde.

3. Verfahren nach Anspruch 1, welches (i) das Markieren der Basophilen in der Vollblutprobe mit anti-IgE-, CD123/HLA-DR- oder CCR3-Antikörpern, (ii) das Hinzufügen vom Mikrokügelchen und (iii) das Analysieren der Probe mittels Durchflusszytometrie umfasst.

4. Verfahren nach Anspruch 2, wobei die mRNA für die α-Kette des hochaffinen IgE-Rezeptors durch Genexpressionsanalyse nach Umkehrtranskription und Erzeugung von cDNA detektiert wird.

5. Verwendung eines Kits für die *In-vitro*-Diagnose von Anaphylaxie unter Verwendung einer Basophilenabsolutzählung, wobei das Kit umfasst:
- Reagenzien zum Färben (Detektion),
- Mikrokügelchen mit bekannter Teilchenzahl,
- Lysepuffer,
- Waschpuffer und
- Gebrauchsanweisung.

6. Verwendung eines Kits für die *In-vitro*-Diagnose von Anaphylaxie unter Verwendung von Expression der α-Kette des hochaffinen IgE-Rezeptors, wobei das Kit umfasst:
- Reagenzien für die Isolation von Gesamt-RNA aus menschlichen Zellen,
- Reagenzien für die cDNA-Umkehrtranskription,
- Primer und Sonden für die α-Kette des hochaffinen IgE-Rezeptorgens (*FCER1A*) und endogene Kontrolle,
- PCR Master Mix,
- Gebrauchsanweisung.

## Revendications

1. Procédé pour le diagnostic de l'anaphylaxie à l'aide d'un compte absolu de basophiles, le procédé comprenant les étapes consistant à :
(1) mesurer un nombre absolu de basophiles dans un échantillon biologique de sang total, qui est prélevé chez un patient jusqu'à 24 h après un épisode d'anaphylaxie présumé ; dans lequel les basophiles sont marqués avec un anticorps apte à se lier spécifiquement aux basophiles et analysés par cytométrie en flux et dans lequel le compte absolu est obtenu par microbilles et
(2) diagnostiquer que le patient a subi une anaphylaxie lorsque le nombre de basophiles mesuré à l'étape (1) est inférieur à un seuil correspondant mesuré avant ou ultérieurement après l'anaphylaxie.

2. Procédé pour le diagnostic de l'anaphylaxie à l'aide de l'expression de la chaîne α d'un récepteur IgE à haute affinité, le procédé comprenant les étapes consistant à :
(1) mesurer un niveau d'expression d'une chaîne α d'un récepteur IgE à haute affinité dans un échantillon biologique de sang total, qui est prélevé chez un patient jusqu'à 24 h après un épisode d'anaphylaxie présumé ; et
(2) diagnostiquer que le patient a subi une anaphylaxie lorsque le niveau d'expression mesuré à l'étape (1) est inférieur à un seuil correspondant mesuré avant ou ultérieurement après l'anaphylaxie.

3. Procédé selon la revendication 1, qui comprend (i) le marquage des basophiles dans l'échantillon de sang total par des anticorps anti-IgE, CD123/HLA-DR ou CCR3, (ii) l'ajout de microbilles, et (iii) l'analyse de l'échantillon par cytométrie en flux.

4. Procédé selon la revendication 2, dans lequel l'ARNm pour une chaîne α d'un récepteur IgE à haute affinité est détecté par analyse d'expression génique après une transcription inverse et une production d'ADNc.

5. Utilisation d'un kit pour le diagnostic *in vitro* de l'anaphylaxie à l'aide d'un compte absolu de basophiles, ledit kit comprenant :
- des réactifs pour colorer (détection),
- des microbilles avec un nombre connu de particules,
- un tampon de lyse,
- un tampon de lavage, et
- un mode d'emploi.

6. Utilisation d'un kit pour le diagnostic *in vitro* de l'anaphylaxie à l'aide d'une expression de la chaîne α d'un récepteur IgE à haute affinité, ledit kit comprenant :
- des réactifs pour l'isolation d'ARN total à partir de cellules humaines,
- des réactifs pour une transcription inverse d'ADNc,
- des amorces et des sondes pour une chaîne α d'un gène récepteur IgE à haute affinité (*FCER1A*) et un témoin endogène,
- du PCR Master Mix,
- un mode d'emploi.
